(19) Europäisches Patentamt
European Patent Office
Office européen des brevets

(11) Publication number: **0 040 364**
**B1**

(12) # EUROPEAN PATENT SPECIFICATION

(45) Date of publication of patent specification: **31.07.85**

(51) Int. Cl.$^4$: **A 61 K 33/42,** A 61 K 35/02

(21) Application number: **81103471.9**

(22) Date of filing: **07.05.81**

(54) **Gastric antacid and method for controlling pH of gastric juice.**

(30) Priority: **14.05.80 JP 62723/80**

(43) Date of publication of application:
**25.11.81 Bulletin 81/47**

(45) Publication of the grant of the patent:
**31.07.85 Bulletin 85/31**

(84) Designated Contracting States:
**DE FR GB IT**

(56) References cited:
**FR-A-2 157 866**
**FR-M- 7 588**
**US-A-3 879 525**

**CHEMICAL ABSTRACTS, vol. 88, no. 26, June 26, 1978, page 407, column 2, abstract 197657r Columbus, Ohio, US**

(73) Proprietor: **KYOWA CHEMICAL INDUSTRY CO., LTD.**
**20, 2-chome Nihonbashi**
**Kayaba-cho Chuo-ku Tokyo (JP)**

(72) Inventor: **Miyata, Shigeo**
**502-3, Yashimanishi-cho**
**Takamatsu-shi Kawaga-ken (JP)**

(74) Representative: **Kraus, Walter, Dr. et al**
**Patentanwälte Kraus Weisert & Partner Thomas-Wimmer-Ring 15**
**D-8000 München 22 (DE)**

**EP 0 040 364 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a new type of gastric antacid which exhibits a rapid and long-lasting buffering action of controlling the pH of gastric juice to an ideal pH range without any likelihood of causing undesirable side-effects such as osteomalacia, or phosphoric-deficient syndrome including hypophosphoremia, hypophosphaturia, hypercalcinuria and anepithymia.

More specifically, this invention relates to a pharmaceutical composition which is characterized by the fact that it contains at least 5% by weight of a phosphate ion-containing hydrotalcite of the following formula

$$Mg_xAl_2(OH)_{2x+6-n_1z_1-n_2z_2}(A_1^{n_1^-})_{z_1}(A_2^{n_2^-})_{z_2} \cdot mH_2O$$

wherein $A_1^{n_1^-}$ represents at least one anion having a valence of $n_1$ selected from the group consisting of $H_2PO_4^-$, $HPO_4^{2-}$ and $PO_4^{3-}$, $A_2^{n_2^-}$ represents at least one anion having a valence of $n_2$ and selected from the group consisting of $CO_3^{2-}$, $(CHOHCOO)_2^{2-}$, $C_6H_5(OH)COO^-$, $C_3H_4OH(COO)_3^{3-}$, $NH_2CH_2COO^-$, $C_5H_7NO_4^{2-}$, $C_3H_6NO_2S^-$, $CH_3CHOHCOO^-$ and $CHOHCH_2(COO)_2^{2-}$, and x, $z_1$, $z_2$ and m are positive numbers satisfying the following expressions

$$2<x<40,$$
$$0<z_1<2,$$
$$0<z_2<2,$$
$$0<m<40,$$
$$0<z_1+z_2\leqq 2,$$

and a pharmaceutically acceptable diluent or carrier.

According to a preferred embodiment of the present invention, the above mentioned composition is in an orally administerable dosage form.

Gastric antacids are used in the treatment of gastric juice including prevention and treatment of gastricisms such as gastritis, gastric ulcer and duodenal ulcer by neutralizing and buffering the pH of gastric juice. The present invention relates to an antacid which is useful in such applications and has an excellent activity.

Antacids are required to rapidly neutralize and buffer the pH of gastric juice to a pH value at which pepsin is inactivated with a long-lasting effect, and should also desirably be stable compounds whose properties do not change over a long period of time and which have a low content of sodium that may cause hypertension.

Aluminium-containing compounds have been used heretofore as antacids. Most typical of these are aluminum hydroxide gel and hydrotalcite [$Mg_6Al_2(OH)_{16}CO_3 \cdot 4H_2O$]. The former is oldest, and is still used most frequently throughout the world. The latter attracted attention as a better antacid than the former, and its use has recently increased.

The increasing acceptance of the latter is due to the fact that it has better stability and has a better action of neutralizing and buffering gastric acid. Aluminum hydroxide gel is an unstable amorphous compound. Immediately after production, its antacid activity is excellent. But since it gradually crystallizes to aluminum hydroxide, its original antacid activity is lost with time. The former also has the defect that because it can control the pH of gastric acid only to a range of about 3 to 4, its activity to inhibit the action of pepsin in gastric juice is not sufficient. In contrast, because hydrotalcite is a stable crystalline compound which is stable to heat, light and water, it has the advantage that even after the lapse of a long period of time from preparation, its antacid activity is not reduced. Furthermore, hydrotalcite can control the pH of gastric acid to an ideal range of about 4 to 6, and therefore, its activity of inactivating pepsin in gastric juice is sufficient, thus bringing about the advantage that the undesirable activity of pepsin on the stomachal wall tissue can be fully inhibited.

It has recently been found however that long-term continued administration of an aluminum-containing antacid causes side-effects resulting in osteomalacia and phosphoric-deficient syndrome including hypophosphoremia, hypophosphaturia, hypercalcinuria and anepithymia. These side-effects occur presumably because an aluminum ion derived from the aluminum-containing antacid and dissolved in gastric juice captures a phosphate ion to be absorbed as calcium phosphate as a main ingredient of the skeleton to form insoluble aluminum phosphate which inhibits absorption of the phosphate ion into the body.

The present inventors made investigations in order to develop a new type of antacid which is free from the aforesaid disadvantages and troubles of the conventional aluminum-containing antacids. These investigations have led to the discovery that hydrotalcite-like compounds containing two kinds of anions ($A_1^{n_1^-}$) and ($A_2^{n_2^-}$) (to be sometimes referred to in the present application as phosphate ion-containing hydrotalcites) conveniently free from the undesirable side-effects of causing the aforesaid phosphoric-deficient syndrome and osteomalacia, and have a rapid and long-lasting activity of neutralizing and buffering the pH of gastric juice to an ideal range of about 4 to about 6; and therefore that they are very

useful for prevention, and treatment of a broad ranges of gastricisms such as gastritis, gastric ulcer and duodenal ulcer while inhibiting the undesirable action of pepsin on the stomachal wall tissue.

The phosphate ion-containing hydrotalcites of formula (1) is accordance with this invention have the advantage that they are very stable to light, heat, water, etc. and for example, have stability to heat of up to about 300°C and on long-term storage, do not decrease in antacid activity owing to aging. Furthermore, these hydrotalcites do not at all cause a side-effect of constipation which is seen in administration of aluminum hydroxide gel nor a lapactic action which is seen in administration of magnesium hydroxide or magnesium oxide presumably because these actions conveniently negate each other in these hydrotalcites. Moreover, since the sodium content of these phosphate ion-containing hydrotalcites is only a trace which can substantially be neglected, they are not likely to cause a side-effect owing to sodium even when administered to persons with hypertension, cardiovascular troubles or renal troubles.

The anions $A_2^{n\bar{2}}$ are inorganic acid ions other than $A_1^{n\bar{1}}$ selected from the group consisting of $CO_3^{2-}$, $(CHOHCOO)_2^{2-}$, $C_6H_5(OH)COO^-$, $C_3H_4OH(COO)_3^{3-}$, $NH_2CH_2COO^-$, $C_5H_7NO_4^{2-}$, $C_3H_6NO_2S^-$, $CH_3CHOHCOO^-$ and $CHOHCH_2(COO)_2^{2-}$.

Anions $A_2$ are $CO_3^{2-}$, tartrate ion $(CHOHCOO)_2^{2-}$, salicylate ion $C_6H_5(OH)COO^-$, citrate ion $C_3H_4OH(COO)_3^{3-}$, glycine ion $NH_2CH_2COO^-$, glutamate ion $C_5H_7NO_4^{2-}$, cystine ion $C_3H_6NO_2S^-$, lactate ion $CH_3CHOHCOO^-$, and malate ion $CHOHCH_2(COO)_2^{2-}$.

Hydrotalcite-like compounds having these two kinds of anions and a process for production thereof are known, and are described, for example in Japanese Patent Publication No. 32198/1972 (U.S. Patent No. 3,879,525, British Patent No. 1,336,865); Japanese Patent Publications Nos. 29477/1973 and 29478/1973; Miyata, Clays and Clay Minerals, Vol. 23, pages 369—375 (1975); and Miyata, Clays and Clay Minerals, Vol. 25, pages 14—18 (1977).

Briefly stated, the phosphate ion-containing hydrotalcite of formula (1) can be produced, for example, by reacting an aluminum compound, preferably a water-soluble aluminum or magnesium compound, with phosphoric acid or its alkali metal or ammonium salt in a liquid medium, preferably in an aqueous medium, under such conditions that the pH of the reaction system is at least about 8. If required, after the reaction, the product may be subjected to various steps such as solvent removal, washing with water, drying, pulverization, and classification.

In another embodiment, a phosphate ion-free hydrotalcite of the following formula

$$Mg_xAl_2(OH)_{2x+6-n_3z_3}(A_3^{n\bar{3}})_{z_3} \cdot mH_2O \qquad (1')$$

wherein x, $n_3$, $z_3$, $A_3^{n\bar{3}}$ and m are as defined with regard to formula (1), $2<x<40$, $0<z_3\leqq 2$, $0<m<40$ is treated with an aqueous solution of an alkali metal or ammonium salt of a phosphate ion ($H_2PO_4^-$, $HPO_4^{2-}$, or $PO_4^{3-}$) or with an aqueous solution of the $A_2^{n\bar{2}}$ ion shown in formula (1) and the aforesaid phosphate ion to exchange a part or the whole of $A_3^{n\bar{3}}$ with the phosphate ion ($A_2^{n\bar{2}}$ may be the same as $A_3^{n\bar{3}}$).

Examples of the aluminum compound used in the first-mentioned embodiment include aluminum halides such as aluminum chloride, aluminum bromide, aluminum iodide and aluminum fluoride; aluminum sulfate; aluminum nitrate; alkali metal aluminates such as sodium aluminate and potassium aluminate; alcohol salts such as aluminum isopropoxide and aluminum ethylate; and basic aluminum salts such as basic aluminum sulfate, basic aluminum chloride and basic aluminum carbonate.

Examples of the magnesium compounds are magnesium halides such as magnesium fluoride, magnesium chloride, magnesium bromide and magnesium iodide; magnesium sulfate; magnesium nitrate; magnesium perchlorate; magnesium salts of organic acids such as magnesium oxalate, magnesium acetate, magnesium citrate and magnesium tartrate; magnesium oxide; and magnesium carbonate.

Examples of phosphoric acid or its alkali metal or ammonium salts include orthophoshporic acid $H_3PO_4$; monoalkali metal hydrogen phosphates such as monosodium hydrogen phosphate $NaH_2PO_4$ and monopotassium hydrogen phosphate; dialkali metal hydrogen phosphates such as disodium hydrogen phosphate $Na_2HPO_4$, dipotassium hydrogen phosphate $K_2HPO_4$; trialkali metal phosphates such as trisodium phosphate $Na_3PO_4$ and tripotassium phosphate $K_3PO_4$; and mono-, di- and tri-ammonium salts of phosphoric acid such as $NH_4H_2PO_4$, $(NH_4)_2HPO_4$, and $(NH_4)_3PO_4$.

In the first-mentioned embodiment, an alkali is used to adjust the pH of the system to at least 8. Examples of the alkali are alkali metal hydroxides such as sodium hydroxide and potassium hydroxide, alkali metal carbonates such as sodium carbonate and potassium carbonate, calcium hydroxide ammonia gas, and aqueous ammonia.

If x in formula (1) is smaller than 2, the antacid activity of the resulting hydrotalcite is insufficient, and if its exceeds 40, the pH buffered by the antacid exceeds about 6. Thus, x is suitably $2<x<40$, preferably about $3\leqq x\leqq 10$, more preferably $4\leqq x\leqq 8$.

In formula (1), each of $A_1^{n\bar{1}}$ and $A_2^{n\bar{2}}$ is not restricted to one kind, but may be a mixture of two or more kinds. As $A_1^{n\bar{1}}$, $HPO_4^{2-}$ is preferred.

In order to have the hydrotalcite of formula (1) exhibit an antacid effect advantageously, $z_1$ is preferably larger than $z_2$. In other words, $A_1^{n\bar{1}}$ is preferably in a major proportion, and $A_2^{n\bar{2}}$, in a minor proportion.

The antacid of this invention may contain various known solid or liquid carriers or diluents and other

additives acceptable for use in antacids, and can be formulated into desired dosage forms with or without various adjuvants known in the field of pharmaceuticals.

Examples of dosage forms are dusts, granules, particles, tablets, coated tablets, suspensions, capsules and other orally administrable forms. The pharmaceutical composition in such a dosage form may contain about 5 to about 95% by weight of the compound of formula (1) based on the total weight of the composition.

If required, the antacid of the invention may further contain another antacid, a vitamin preparation, an anti-inflammatory agent, a cardiac agent, an anti-hypertensive agent, an analgesic agent, an antihistaminic agent, an antipyretic-analgesic agent, a digestive, or an antitussive agent. Or it may be used in combination with these agents or other drugs.

The dose of the antacid of the invention can be properly selected depending upon the purpose of administration or the condition of the patient, but may be within the effective range of conventional antacids. For example, the dose is about 10 to about 100 mg/day/kg of body weight as the active component (1) in oral administration.

The antacid of the invention is a useful and unique antacid for prevention and treatment of gastritis, chlorhydria, gastric ulcer, and other similar gastric troubles, and exhibits a better action than conventional antacids without involving the side-effects of the conventional antacids.

The following Examples illustrate the present invention more specifically.

Example 1

Five liters of a mixed aqueous solution of aluminum chloride and magnesium chloride ($Al^{3+}$=0.2 mole/liter, $Mg^{2+}$=0.8 mole/liter) and 5 liters of an aqueous solution of disodium hydrogen phosphate $Na_2HPO_4$ (0.1 mole/liter) were added at a flow rate of 200 ml/min by means of a metering pump to a 5-liter cylindrical stainless steel reactor fitted with a stirrer and a pH meter together with a 3 mole/liter aqueous solution of sodium hydroxide. The pH of the reaction mixture was maintained at about 9 to 10, and the reaction was performed continuously. The resulting white precipitate was filtered under reduced pressure, washed fully with water, and dried at about 100°C for 10 hours. The dried product had the following properties.

X-ray diffraction pattern: identified as hydrotalcite crystals
Chemical composition: $Mg_8Al_2(OH)_{19.5}(HPO_4)_{0.87} \cdot (CO_3)_{0.38} \cdot 5.5H_2O$
Sodium content: 0.006%.

The antacid activity of the product was examined by the following testing methods, and the results are shown in Table 1.

[Antacid activity test].

Fuch's test

Fifty milliliters of 0.1-normal hydrochloric acid was put into a 300 ml beaker, and the beaker was dipped in a constant temperature tank kept at 37±1°C. The electrode of the pH meter was put into it, and the HCl was stirred by a magnetic stirrer. When its temperature reached 37°C, 0.5 g of a sample was put into the beaker. Ten minutes after the addition of the sample, 0.1-normal hydrochloric acid was added continuously by means of a metering pump at a rate of 2 ml/min until the pH of the mixture reached 3 or below. Variations in pH were continuously recorded automatically.

pH Stat test

Water (20 ml) was put into a beaker having a volume of about 50 ml. The beaker was put into a constant temperature tank set at 37±1°C. The electrode of the pH meter was put into the beaker, and the water was stirred by a magnetic stirrer. The pH meter was interlockingly connected to a pH stat device. The pH was set at 3.0, and 200 mg of a sample was added. To maintain the pH of this system at 3.0, 1-normal hydrochloric acid was automatically fed to the beaker. The pH and the amount of 1-normal hydrochloric acid fed were automatically recorded as a function of time, and the time which elapsed until 50% and 80% respectively of the sample dissolved in 1-normal hydrochloric acid was measured. The shorter the time measured, the more rapid the reaction of the antacid with the acid.

Example 2

Five liters of a mixed aqueous solution of magnesium nitrate and aluminum nitrate ($Mg^{2+}$=0.6 mole/liter, $Al^{3+}$=0.2 mole/liter) and a 4 mole/liter aqueous solution of sodium hydroxide were added to a 5-liter cylindrical stainless steel reactor being stirred with a stirrer and containing the electrode of a pH meter dipped therein. The pH of the system was adjusted to 10 to 10.5 by the aqueous solution of sodium hydroxide. The reaction was continuously performed at room temperature. The resulting white precipitate was hydrotalcite having the composition $Mg_6Al_2(OH)_{16}(NO_3)_{1.6}(CO_3)_{0.1} \cdot 2.3H_2O$.

Five hundred grams, calculated as the dry product, of the suspension containing this precipitate was taken, filtered under reduced pressure, and then washed with about 6.5 liters of a 0.2 mole/liter aqueous

solution of disodium hydrogen phosphate to ion-exchange $NO_3$ with $HPO_4^{2-}$. The product was washed with water, dehydrated and dried. Chemical analysis of the product showed the following results.

Chemical composition: $Mg_6Al_2(OH)_{15.4}(HPO_4)_{0.84}(CO_3)_{0.46} \cdot 3.74H_2O$
Sodium content: 0.002%
X-ray diffraction pattern: identified as hydrotalcite crystals.

The antacid activity of this product is shown in Table 1.

Example 3

A mixed aqueous solution of magnesium chloride and aluminum chloride ($Mg^{2+}$=0.5 mole/liter, $Al^{3+}$=0.2 mole/liter) and a 4.0 mole/liter aqueous solution of sodium hydroxide were reacted in the same way as in Example 2 to give a hydrotalcite of the formula $Mg_5Al_2(OH)_{14}Cl_2 \cdot 3.2H_2O$.

Five hundred grams of the resulting hydrotalcite was washed with 4 liters of a 0.1 mole/liter aqueous solution of trisodium phosphate to ion-exchange Cl by $PO_4^{3-}$. The product was then washed with water and dried to give a product having the following properties.

Chemical composition: $Mg_5Al_2(OH)_{13.67}(PO_4)_{0.71}(CO_3)_{0.10} \cdot 3.1H_2O$
Sodium content: 0.003%
X-ray diffraction pattern: identified as hydrotalcite crystals
The antacid activity of the product is shown in Table 1.

Example 4

A hydrotalcite of the following formula

$$Mg_4Al_2(OH)_{12}(Cl)_{1.6}(CO_3)_{0.2} \cdot 1.2H_2O$$

was prepared by reacting a mixed aqueous solution of aluminum chloride and magnesium chloride ($Al^{3+}$=0.3 mole/liter, $Mg^{2+}$=0.6 mole/liter) and a 3 mole/liter aqueous solution of sodium hydroxide in the same way as in Example 2.

Five hundred grams of this hydrotalcite was washed with 4 liters of 0.4 mole/liter monosodium hydrogen phosphate and subsequently with 4 liters of 0.2 mole/liter of sodium carbonate to perform ion exchange. The product was washed with water and dried. The product had the following properties.

Chemical composition: $Mg_4Al_2(OH)_{12}(H_2PO_4)_{0.7}(CO_3)_{0.6} \cdot 0.8H_2O$
Sodium content: 0.008%
X-ray diffraction pattern: identified as hydrotalcite.

The antacid activity of the product is shown in Table 1.

TABLE 1

| Example | Fuch's | | | | pH Stat test | |
|---|---|---|---|---|---|---|
| | Time required until the pH reacted 3 (seconds) | Time required until the pH reached 3.5 (seconds) | Maximum pH | Time required until the pH was buffered to more than 3 (minutes) | T50 (the time required until 50% was reacted) (seconds) | T80 (the time required until 80% was reacted) (seconds) |
| 1 | 3 | 5 | 5.5 | 84 | 30 | 80 |
| 2 | 5 | 7 | 4.8 | 79 | 100 | 260 |
| 3 | 8 | 10 | 4.1 | 77 | 230 | 435 |
| 4 | 12 | 19 | 4.2 | 86 | 90 | 176 |

**Claims**

1. A pharmaceutical composition characterized by containing at least 5% by weight of a phosphate ion-containing hydrotalcite of the following formula

$$Mg_xAl_2(OH)_{2x+6-n_1z_1-n_2z_2}(A_1^{n_1-})_{z_1}(A_2^{n_2-})_{z_2}\cdot mH_2O$$

wherein $A_1^{n_1-}$ represents at least one anion having a valence of $n_1$ selected from the group consisting of $H_2PO_4^-$, $HPO_4^{2-}$ and $PO_4^{3-}$, $A_2^{n_2-}$ represents at least one anion having a valence of $n_2$ and selected from the group consisting of $CO_3^{2-}$, $(CHOHCOO)_2^{2-}$, $C_6H_5(OH)COO^-$, $C_3H_4OH(COO)_3^{3-}$, $NH_2CH_2COO^-$, $C_5H_7NO_4^{2-}$, $C_3H_6NO_2S^-$, $CH_3CHOHCOO^-$ and $CHOHCH_2(COO)_2^{2-}$, and x, $z_1$, $z_2$ and m are positive numbers satisfying the following expressions

$$2<x<40,$$
$$0<z_1<2,$$
$$0<z_2<2,$$
$$0<m<40,$$
$$0<z_1+z_2\leqq 2,$$

and a pharmaceutically acceptable diluent or carrier.

2. The composition of claim 1 which is in an orally administerable dosage form.

**Patentansprüche**

1. Arzneimittel, dadurch gekennzeichnet, daß es mindestens 5 Gew.-% eines Phosphationen enthaltenden Hydrotalcits der folgenden Formel:

$$Mg_xAl_2(OH)_{2x+6-n_1z_1-n_2z_2}(A_1^{n_1-})_{z_1}(A_2^{n_2-})_{z_2}\cdot mH_2O$$

worin $A_1^{n_1-}$ für mindestens ein Anion mit einer Wertigkeit von $n_1$, ausgewählt aus der Gruppe, bestehend aus $H_2PO_4^-$, $HPO_4^{2-}$ und $PO_4^{3-}$, steht, $A_2^{n_2-}$ für mindestens ein Anion mit einer Wertigkeit von $n_2$ und ausgewählt aus der Gruppe, bestehend aus $CO_3^{2-}$, $(CHOHCOO)_2^{2-}$, $C_6H_5(OH)COO^-$, $C_3H_4OH(COO)_3^{3-}$, $NH_2CH_2COO^-$, $C_5H_7NO_4^{2-}$, $C_3H_6NO_2S^-$, $CH_3CHOHCOO^-$ und $CHOHCH_2(COO)_2^{2-}$, steht und x, $z_1$, $z_2$ und m positive Zahlen sind, die den folgenden Bedingungen:

$$2<x<40,$$
$$0<z_1<2,$$
$$0<z_2<2,$$
$$0<m<40,$$
$$0<z_1+z_2\leqq 2,$$

genügen, und ein pharmazeutisch annehmbares Verdünnungsmittel oder Träger enthält.

2. Arzneimittel nach Anspruch 1, dadurch gekennzeichnet, daß es in einer oral verabreichbaren Dosierungsform vorliegt.

**Revendications**

1. Composition pharmaceutique caractérisée en ce qu'elle contient au moins 5% en poids d'une hydrotalcite contenant un ion phosphate et de formule suivante:

$$Mg_xAl_2(OH)_{2x+6-n_1z_1-n_2z_2}(A_1^{n_1-})_{z_1}(A_2^{n_2-})_{z_2}\cdot mH_2O$$

dans laquelle $A_1^{n_1-}$ représente au moins un anion de valence $n_1$ choisi dans le groupe consistant en $H_2PO_4^-$, $HPO_4^{2-}$ et $PO_4^{3-}$; $A_2^{n_2-}$ représente au moins un anion de valence $n_2$ choisi dans le groupe consistant en $CO_3^{2-}$, $(CHOHCOO)_2^{2-}$, $C_6H_5(OH)COO^-$, $C_3H_4OH(COO)_3^{3-}$, $NH_2CH_2COO^-$, $C_5H_7NO_4^{2-}$, $C_3H_6NO_2S^-$, $CH_3CHOHCOO^-$ et $CHOHCH_2(COO)_2^{2-}$; et x, $z_1$, $z_2$ et m sont des nombres positifs vérifiant les relations suivantes:

$$2<x<40,$$
$$0<z_1<2,$$
$$0<z_2<2,$$
$$0<m<40,$$
$$0<z_1+z_2\leqq 2,$$

et un diluant ou support pharmaceutiquement acceptable.

2. Composition selon la revendication 1, qui est sous une forme dosée pouvant être administrée oralement.